# EUROPEAN PATENT APPLICATION

(11) **EP 3 714 730 A1**
(43) Date of publication of application: **30.09.2020**
(21) Application number: 19166397.0
(22) Date of filing: 29.03.2019
(51) Int. Cl.: A45D 34/02, A61M 5/315, A61M 5/00, A61M 5/50

(54) **CARTRIDGE CONTAINING A PERFUME CONCENTRATE**

(71) Applicant: My Scent Pte Ltd, Singapore 188778 (SG)
(72) Inventor: MONANGE, Johanna, 188778 SINGAPORE (SG)
(74) Representative: den Braber, Gérard Paul

(57) **Abstract**

A cartridge (100) contains a perfume concentrate (101). The cartridge (100) comprises a syringe (102) in which the perfume concentrate (101) is contained. The syringe (102) comprises a barrel (103) having an outlet (104) and a plunger (105). The cartridge (101) defines a threshold force that needs to be exceeded for pushing the plunger (105) towards the outlet (104). In some embodiments, the syringe (102) essentially comprises at least one of the following materials: polypropylene, polyurethane, and glass

## Description

### FIELD OF THE INVENTION

An aspect of the invention relates to a cartridge containing a perfume concentrate. The cartridge may be used, for example, to prepare a personalized perfume composition. Further aspects of the invention relate to a perfume preparation assembly and a perfume preparation apparatus.

### BACKGROUND ART

Patent publication GB191222745A describes that perfumes have been sold in a diluted condition ready for use and generally contained in glass bottles of a more or less ornate character. Under such circumstances the cost to the user of perfumes, is unduly enhanced by that of the container of the perfume and the cost of transport thereof. The true value of the perfume resides almost entirely in that of the essence contained therein, which constitutes a relatively small fraction of the total mass, bought and sold.

The whole cost of the vehicle and the charges on its transport can practically be eliminated by supplying the essence in a concentrated condition contained within an impermeable envelope. To render the perfume available for use, the envelope is adapted to be dissolved, penetrated or broken after insertion within a bottle or other container and diluted by the addition of alcohol or other suitable volatile liquid.

For example, the perfume essence may be enclosed within a capsule of gelatine which, after insertion by the purchaser within a bottle can be dissolved by the addition of: a small quantity of hot water. Alternatively the essence may be contained within a rubber envelope or within tubes of glass or within metal foil. Whatever envelope is adapted it must be capable of being hermetically sealed and readily destroyed, broken or penetrated when desired by the user of the perfume to permit the diluting liquid to be intimately intermixed with the concentrated essence.

### SUMMARY OF THE INVENTION

There is a need for an improved solution for customized perfume preparation on the basis of perfume concentrate that better meets at least one of the following criteria: convenience of use and safety.

In accordance with an aspect of the invention as defined in claim 1, there is provided a cartridge containing a perfume concentrate. The cartridge comprises a syringe in which the perfume concentrate is contained. The syringe comprises a barrel having an outlet and a plunger. The cartridge defines a threshold force that needs to be exceeded for pushing the plunger towards the outlet.

The perfume concentrate, which is contained in the syringe, can conveniently and precisely be transferred into a bottle, which reduces risk of spilling. There is also a reduced risk of perfume concentrate accidentally leaving the cartridge because a threshold force needs to be exceeded for pushing the plunger towards the outlet. This contributes to safety because a perfume concentrate may be relatively corrosive and, when not diluted, cause irritation of biological tissue. In some embodiments, the syringe essentially comprises at least one of the following materials: polypropylene, polyurethane, and glass.

In accordance with further aspects of the invention as defined in claims 13 and 14 there are provided a perfume preparation assembly and a perfume preparation apparatus, respectively.

By way of illustration, some embodiments of the invention are described in detail with reference to accompanying drawings. In this description, additional features will be presented, some of which are defined in the dependent claims, and advantages will be apparent.

### BRIEF DESCRIPTION OF THE DRAWINGS

FIG. 1 is a schematic perspective view of a cartridge containing a perfume concentrate.
FIG. 2 is a schematic front view of the cartridge.
FIG. 3 is a schematic cross sectional view of the cartridge along a cut line A-A indicated in FIG. 2.
FIG. 4 is a schematic perspective view of a tubular body that forms part of the cartridge.
FIG. 5 is a schematic front view of a plunger of a syringe comprised in the cartridge.
FIG. 6 is an enlarged schematic front view of a portion of the plunger.
FIG. 7 is a schematic perspective view of a cap that is removably fitted on the tubular body.
FIG. 8 is a schematic front view of a perfume preparation assembly in an uncoupled state.
FIG. 9 is a schematic front view of a perfume preparation assembly in a coupled state.
FIG. 10 is a schematic block diagram of a perfume preparation apparatus

### DESCRIPTION OF SOME EMBODIMENTS

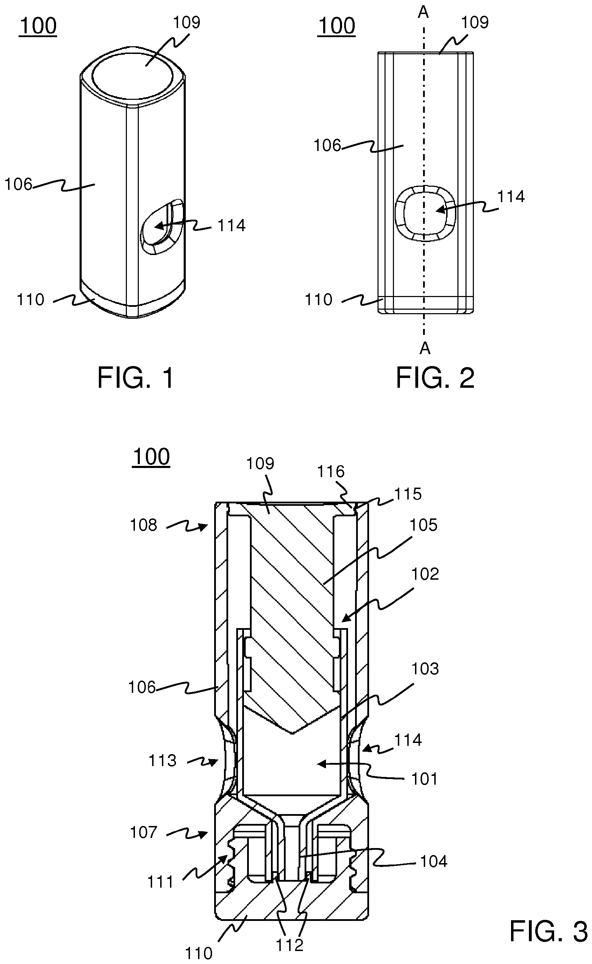

FIGS. 1-3 schematically illustrate a cartridge 100 containing a perfume concentrate 101. FIG. 1 provides a schematic perspective view of the cartridge 100. FIG. 2 provides a schematic front view of the cartridge 100. FIG. 3 provides a schematic cross-sectional view of the cartridge 100 along a cut line A-A indicated in FIG. 2.

The perfume concentrate 101 in the cartridge may comprise, for example, a mix of different natural essences prepared by a perfumer. Alternatively, the perfume concentrate 101 may comprise a single essence. The perfume concentrate 101 may further comprise one or more synthetic molecules. The perfume concentrate 101 is adapted to be diluted into an appropriate dilution fluid such as, for example, alcohol. Accordingly, a consumer may prepare, for example, a cologne or a perfume in French referred to as "eau de toilette" or "eau de parfum", respectively.

The cartridge 100 comprises a syringe 102 in which the perfume concentrate 101 is contained as illustrated in FIG. 3. The syringe 102 comprises a barrel 103 having an outlet 104 and a plunger 105. The plunger 105 may also be designated by the term "piston". In the sequel, the term plunger 105 will continued to be used.

The syringe 102 essentially comprises at least one of the following materials: polypropylene, polyurethane, and glass. These materials have been found to be sufficiently resistant to numerous different perfume concentrates, some of which may be relatively corrosive. In some embodiments, polypropylene or polyurethane may be preferred for reasons related to, for example, cost. The syringe may have a length that is in the order of centimeters, and a diameter that is also in the order of centimeters, the diameter being typically smaller than the length.

The cartridge 100 further comprises a tubular body 106 that surrounds the syringe 102. The barrel 103 abuts against the tubular body 106 at an end 107 where the outlet 104 is present. This end 107 of the tubular body 106 will hereinafter be referred to as lower end 107 for the sake of convenience. Another end 108 of the tubular body 106, which is opposite to the lower end 107, will hereinafter be referred to as upper end 108 for the sake of convenience. An exposed disk-shaped end part 109 of the plunger 105 closes off the upper end 108 of the tubular body 106.

The cartridge 100 further comprises a removable cap 110 at the lower end 107 of the tubular body 106. The removable cap 110 is coupled to the tubular body 106 by means of a screw connection 111. The screw connection 111 comprises a threaded section in the lower end 107 of the tubular body 106. The removable cap 110 comprises a complementary threaded section that can engage with the threaded section in the lower end 107 of the tubular body 106. The removable cap 110 further comprises an inwardly protruding circular rim 112 that engages with an end portion of the outlet 104 of the syringe 102.

The tubular body 106 comprises two openings 113, 114 that expose at least a part of the syringe 102. In case this part of the syringe 102 is transparent, such an opening may make the perfume concentrate 101 visible. The perfume concentrate 101 may have a specific color that allows distinguishing between various cartridges similar to the cartridge 100 illustrated in FIGS. 1-3, which contain different perfume concentrates.

The tubular body 106 comprises a protrusion 115, which is located at the upper end 108 in this embodiment. The plunger 105 comprises a notch 116, which, in this embodiment, is present in an outer circumferential edge of the exposed disk-shaped end part 109 of the plunger 105. The protrusion 115 of the tubular body 106 is engaged with the notch 116 in the plunger 105. Accordingly, the plunger 105 is releasably locked to the tubular body 106.

In order to unlock the plunger 105 from the tubular body 106, a pushing force that exceeds a threshold force needs to be exerted on the plunger 105. In this embodiment, the protrusion 115 and the notch 116 define this threshold force. Once the plunger 105 is released, the plunger 105 may move toward the outlet 104 assuming that the removable cap 110 has been removed. The perfume concentrate 101 will then flow out of the syringe 102 through the outlet 104, and thus flow out of the cartridge 100.

In an alternative embodiment, a plunger may comprise a protrusion and the tubular body may comprise a notch. This is a structural inverse of the embodiment illustrated in FIGS. 1-3. The protrusion of the plunger may engage with the notch in the tubular body. Accordingly, in the alternative embodiment too, the plunger is releasably locked to the tubular body. The protrusion and the notch define a threshold force that needs to be exceeded in order to unlock the plunger from tubular body.

More generally, the embodiment of the cartridge 100 illustrated in FIGS. 1-3, as well as the alternative embodiment, defines a threshold force that needs to be exceeded for pushing the plunger 105 towards the outlet 104. The same can be achieved in numerous alternative manners.

For example, in another alternative embodiment, a tubular body surrounding a syringe may comprise a sealing cap on an end where a plunger is present. In order to be able to access and push the plunger, the sealing cap needs to be broken or, alternatively, removed. The sealing cap, which forms part of this alternative cartridge, thus defines a threshold force that needs to be exceeded to break or remove the sealing cap so as to access and push the plunger.

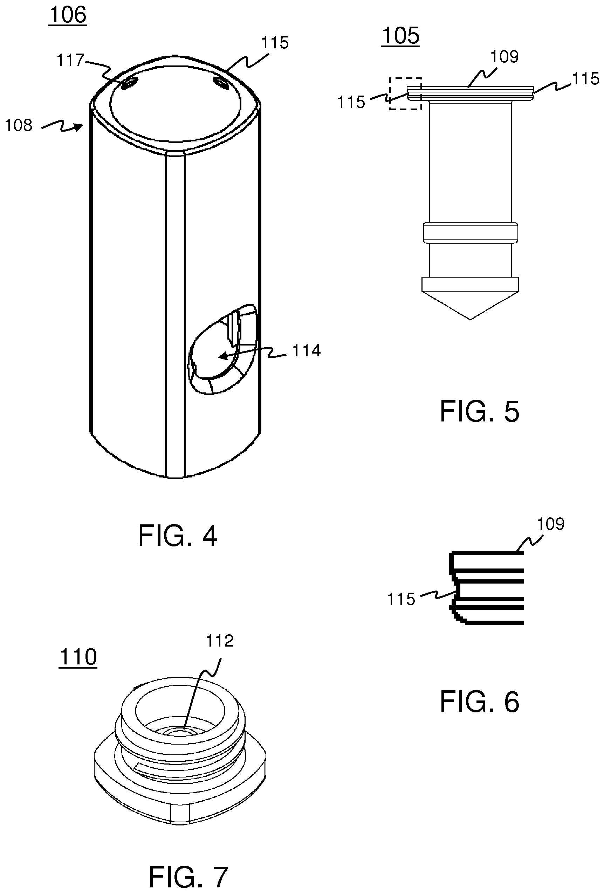

FIG. 4 schematically illustrates the tubular body 106 that forms part of the cartridge 100 illustrated in FIGS. 1-3 and described hereinbefore. FIG. 4 provides a schematic perspective view of the tubular body 106. FIG. 4 illustrates that the protrusion 115 indicated in FIG. 3 is one of a plurality of protrusions 115, 117. The protrusions 115, 117 extend from an inner surface of the tubular body 106 at its upper end 108.

FIG. 5 schematically illustrates the plunger 105 of the syringe 102. FIG. 5 provides a schematic front view of the plunger 105 comprised in the cartridge 100. The notch 116 in the outer circumferential edge of the exposed disk-shaped end part 109 of the plunger 105 is visible. FIG. 5 illustrates that this notch 116 is circumferential, forming a circumferential groove in the outer circumferential edge.

FIG. 6 schematically illustrates a portion of the outer circumferential edge of the exposed disk-shaped end part 109 of the plunger 105. FIG. 6 provides an enlarged schematic front view of this portion, which is indicated by means of a broken-line rectangle in FIG.5.

FIG. 7 schematically illustrates the removable cap 110 of the cartridge 100 illustrated in FIGS. 1-3 and described hereinbefore. FIG. 7 provides a schematic perspective view of the removable cap 110. The complementary threaded section of the removable cap 110 mentioned hereinbefore with reference to FIG. 3 is visible. A portion of the inwardly protruding circular rim 112 of the removable cap 110 is also visible.

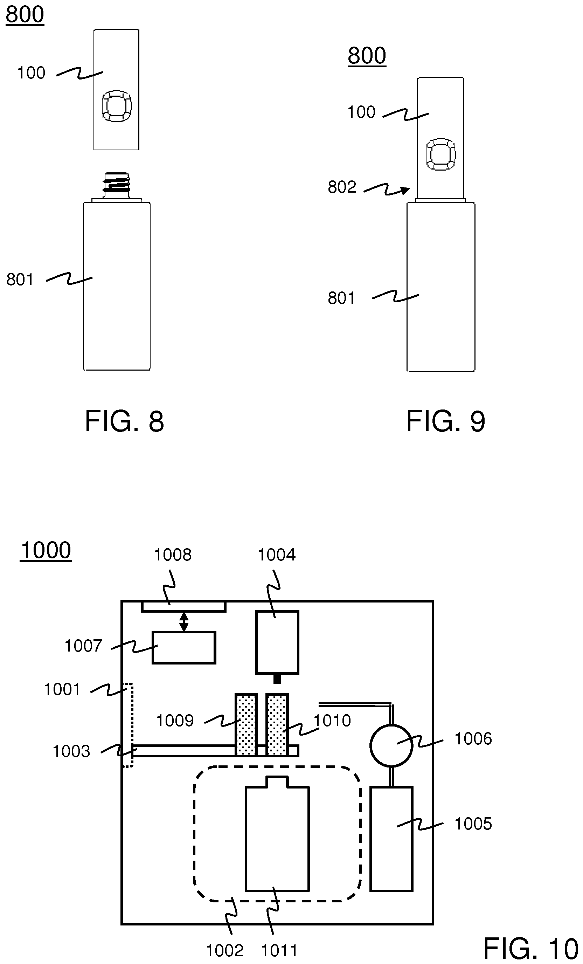

FIG. 8 schematically illustrates a perfume preparation assembly 800 in an uncoupled state. FIG. 8 provides a schematic front view of the perfume preparation assembly 800 in this state. The perfume preparation assembly 800 comprises the cartridge 100 illustrated in FIGS. 1-3 and described hereinbefore. The perfume preparation assembly 800 further comprises a bottle 801.

FIG. 9 schematically illustrates the perfume preparation assembly 800 in a coupled state. FIG. 9 provides a schematic front view of the perfume preparation assembly 800 in this state where the cartridge 100 is coupled to the bottle 801. More specifically, the cartridge 100 is releasably engaged with the bottle 801. The cartridge 100 may be releasably engaged with the bottle 801 by means of, for example, a snap connection 802.

Referring also to FIG. 1, in the coupled state, the perfume concentrate 101 contained in the syringe 102 can flow into the bottle 801 when the plunger 105 of the syringe 102 is pushed towards the outlet 104. To that end, a user may place a finger on the exposed disk-shaped end part 109 of the plunger 105. The user may then exert a pushing force on this part that exceeds the aforementioned threshold force. Once a desired quantity of the perfume concentrate 101 has been released into the bottle 801, the cartridge 100 can disengage from the bottle 801. The perfume preparation assembly 800 then returns to the uncoupled state illustrated in FIG. 8.

In a perfume preparation method, another cartridge may subsequently be coupled to the bottle 801, which already contains the perfume concentrate 101 that was present in the cartridge 100 illustrated in FIG. 1. The other cartridge may comprise another perfume concentrate, which can be released into the bottle 801 in a manner as described hereinbefore. Accordingly, different perfume concentrates may conveniently and safely be introduced into the bottle 801, if so desired. Thereafter, a diluting fluid, such as, for example, ethanol can be introduced. Accordingly, a personalized perfume composition can be prepared.

FIG. 10 schematically illustrates a perfume preparation apparatus 1000. FIG. 10 provides a schematic block diagram of the perfume preparation apparatus 1000. The perfume preparation apparatus 1000 comprises a cartridge introduction port 1001, a bottle receiving space 1002, a cartridge transporting arrangement 1003, a cartridge emptying arrangement 1004, a dilution fluid container 1005, a fluid pumping and transportation arrangement 1006, a controller 1007, and a user interface 1008.

The perfume preparation apparatus 1000 basically operates as follows. A user may introduce a cartridge into the perfume preparation apparatus 1000 through the cartridge introduction port 1001. The perfume preparation apparatus 1000 may comprise several such cartridges 1009, 1010 that have been introduced. The cartridges 1009, 1010 may be similar to the cartridge 100 illustrated in FIGS. 1-3 and described hereinbefore. The user may further place a bottle 1011 in the bottle receiving space 1002, which may be an opening in a front face of the perfume preparation apparatus 1000. The user may further ensure that a sufficient quantity of dilution fluid, such as, for example, ethanol, is present in the dilution fluid container 1005.

Once these preparatory operations have been completed, the user may select at least one cartridge among the cartridges 1009, 1010 that have been introduced in the machine. The cartridges 1009, 1010 may comprise a machine-readable indication specifying a perfume concentrate that the cartridge contains. In that case, the perfume preparation apparatus 1000 may display the perfume concentrates that are available through the user interface 1008. The user may then select at least one perfume concentrate among these available perfume concentrates. In case the user selects various perfume concentrates, the user may further specify a ratio between these selected concentrates. In addition, the user may specify a degree of dilution, which may correspond with a ratio between a quantity of perfume concentrate and a quantity of dilution fluid.

Once these selection operations have been completed, the controller 1007 may cause the cartridge transporting arrangement 1003 to transport a selected cartridge so that the selected cartridge is operable by the cartridge emptying arrangement 1004. For the sake of explanation, it is assumed that the selected cartridge is the cartridge 100 illustrated in FIGS. 1-3 and described hereinbefore. Referring also to FIG. 1, the controller 1007 then causes the cartridge emptying arrangement 1004 to push the perfume concentrate 101 out of the syringe 102 and into the bottle 1011. The cartridge emptying arrangement 1004 may comprise, for example, a piston that exerts a pushing force on the exposed disk-shaped end part 109 of the plunger 105. The pushing force exceeds the threshold force mentioned hereinbefore.

Once a desired quantity of selected perfume concentrates has been introduced into the bottle 1011, the controller 1007 may cause the fluid pumping and transportation arrangement 1006 to introduce a desired quantity of dilution fluid into the bottle 1011. After this, the bottle 1011 comprises a perfume composition, which may be ready for use. The bottle 1011 may then be taken out of the perfume preparation apparatus 1000 and may be closed with a cap.

### NOTES

The embodiments described hereinbefore with reference to the drawings are presented by way of illustration. The invention may be implemented in numerous different ways. In order to illustrate this, some alternatives are briefly indicated.

There are numerous different ways of implementing a cartridge in accordance with the invention. In the present embodiments, a cap is fitted on a tubular body. In other embodiments, a tubular body may comprise a seal that may be broken or that may be penetrated by an outlet of a syringe, for example, by moving the syringe into tubular body.

The term "bottle" should be understood in a broad sense. The term may embrace any entity that is capable of containing a perfume concentrate, which may be diluted or not.

In general, there are numerous different ways of implementing the invention, whereby different implementations may have different topologies. In any given topology, a single entity may carry out several functions, or several entities may jointly carry out a single function. In this respect, the drawings are very diagrammatic.

The remarks made hereinbefore demonstrate that the embodiments described with reference to the drawings illustrate the invention, rather than limit the invention. The invention can be implemented in numerous alternative ways that are within the scope of the appended claims. All changes that come within the meaning and range of equivalency of the claims are to be embraced within their scope. Any reference sign in a claim should not be construed as limiting the claim. The verb "comprise" in a claim does not exclude the presence of other elements or other steps than those listed in the claim. The same applies to similar verbs such as "include" and "contain". The mention of an element in singular in a claim pertaining to a product, does not exclude that the product may comprise a plurality of such elements. Likewise, the mention of a step in singular in a claim pertaining to a method does not exclude that the method may comprise a plurality of such steps. The mere fact that respective dependent claims define respective additional features, does not exclude combinations of additional features other than those reflected in the claims.

## Claims

1. A cartridge (100) containing a perfume concentrate (101), wherein the cartridge comprises a syringe (102) in which the perfume concentrate is contained, the syringe comprising a barrel (103) having an outlet (104) and a plunger (105), the cartridge defining a threshold force that needs to be exceeded for pushing the plunger towards the outlet.

2. A cartridge according to claim 1, wherein the syringe (102) essentially comprises at least one of the following materials: polypropylene, polyurethane, and glass.

3. A cartridge according to any of claims 1 and 2, wherein the cartridge comprises a tubular body (106) that surrounds the syringe, whereby the barrel (103) abuts against the tubular body at an end (107) where the outlet (104) is present.

4. A cartridge according to claim 3, wherein the plunger (105) is releasably locked to the tubular body (106), whereby a pushing force exerted on the plunger that exceeds threshold force causes the plunger to unlock from the tubular body and move toward the outlet (104).

5. A cartridge according to claim 4, wherein the tubular body (106) comprises a protrusion (115) and the plunger (105) comprises a notch (116), the protrusion of the tubular body being adapted to engage with the notch in the plunger so that the plunger is releasably locked to the tubular body.

6. A cartridge according to claim 4, wherein the plunger comprises a protrusion and the tubular body comprises a notch, the protrusion of the plunger being adapted to engage with the notch in the tubular body so that the plunger is releasably locked to the tubular body.

7. A cartridge according to any of claims 5 and 6, wherein the notch (116) is circumferential, and the protrusion (115) is one of a plurality of protrusions (115, 117) adapted to engage with the notch.

8. A cartridge according to any of claims 1 to 7, wherein the cartridge is adapted to releasably engage with a bottle (801) so that the perfume concentrate (101) contained in the syringe (102) can flow into the bottle when the plunger (105) of the syringe is pushed towards the outlet (104), where after the cartridge can disengage from the bottle.

9. A cartridge according to claim 8, wherein the cartridge is adapted to releasably engage the bottle (801) by means of a snap connection (802).

10. A cartridge according to any of claims 3 to 9, wherein the cartridge comprises a removable cap (110) at the end (107) of the tubular body (106) where the outlet (104) of the syringe (102) is present.

11. A cartridge according to claim 10, wherein the removable cap (110) is coupled to the tubular body (106) by means of a screw connection (111).

12. A cartridge according to any of claims 9 to 11, wherein the tubular body (106) comprises an opening (113, 114) exposing at least a part of the syringe (102).

13. A perfume preparation assembly (800) comprising a bottle (801) and a cartridge (100) according to any of claims 1 to 12.

14. A perfume preparation apparatus (1000) adapted to operate on a cartridge (100) according to any of claims 1 to 12 so as to push the perfume concentrate (101) out of the syringe (102) and into a bottle (1011).

15. A perfume preparation apparatus according to claim 13, wherein the perfume preparation apparatus is adapted to introduce into the bottle (1011) a fluid in which the perfume concentrate (101) dilutes.
